# EUROPEAN PATENT SPECIFICATION

(11) **EP 4 140 405 B1**
(45) Date of publication and mention of the grant of the patent: **05.06.2024**
(21) Application number: 21791822.6
(22) Date of filing: 08.04.2021
(51) Int. Cl.: A61B 5/097, A61B 5/08, A61B 5/00, A61B 10/00, G01N 1/24, G01N 33/497, G01N 1/22

(54) **LOOP-TYPE SAMPLING DEVICE AND BREATHALYZER COMPRISING SAME**
SCHLEIFENARTIGE PROBENAHMEVORRICHTUNG UND ATEMANALYSATOR DAMIT
DISPOSITIF D'ÉCHANTILLONNAGE DE TYPE BOUCLE ET ÉTHYLOMÈTRE LE COMPRENANT

(30) Priority: 24.04.2020 KR 20200050260
(43) Date of publication of application: 01.03.2023
(73) Proprietor: Sentech Korea Corp., Paju-si, Gyeonggi-do 10880 (KR)
(72) Inventor: YOO, Do Joon, Seongnam-si, Gyeonggi-do 13456 (KR); LEE, Jun No, Goyang-si, Gyeonggi-do 10306 (KR); LEE, Tae Woo, Goyang-si, Gyeonggi-do 10222 (KR); KIM, Yong, Goyang-si, Gyeonggi-do 10370 (KR); CHOI, Kwang Hyun, Goyang-si, Gyeonggi-do 10412 (KR)
(74) Representative: v. Bezold & Partner Patentanwälte - PartG mbB
(86) International application number: PCT/KR2021/004388
(87) International publication number: WO 2021/215711

(56) References cited:
- EP-A1- 3 059 572
- EP-A1- 3 059 572
- KR-A- 20110 118 816
- KR-B1- 101 873 642
- KR-B1- 101 998 612
- US-A1- 2019 154 848
- US-B2- 7 051 605

## Description

### Technical Field

The present disclosure relates to a loop-type sampling device and a breathalyzer including the same. More particularly, the present disclosure relates to a loop-type sampling device and a breathalyzer including the loop-type sampling device that is capable of minimizing an infection caused by droplets included in an exhalation (exhaled gas) of a subject.

### Background Art

A breathalyzer measures a concentration of alcohol that is exhaled together with an exhalation of a subject. Since the concentration of alcohol discharged together with the exhalation is proportional to a concentration of alcohol in blood, a blood alcohol concentration (BAC) is capable of being calculated when the concentration of alcohol contained in the exhalation is measured.

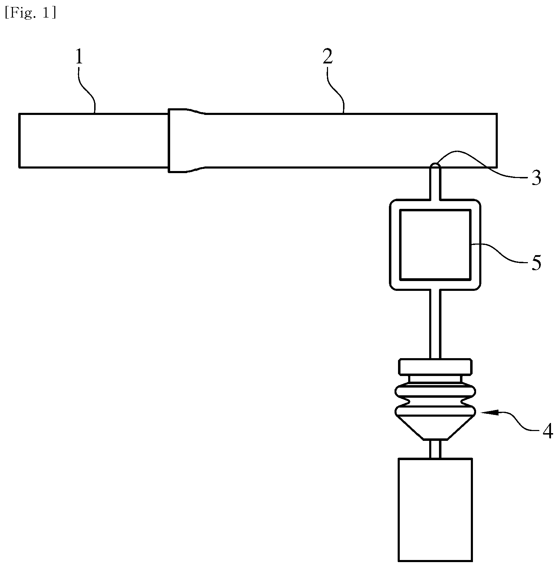

As illustrated in FIG. 1, in a conventional breathalyzer, an exhalation passage 2 formed in a cylindrical shape and coupled to a blowing tube 1 is used as a sampling device for collecting a part of the exhalation. The exhalation passage 2 has both ends that are open. The blowing tube 1 may be coupled to an inlet port of a first end of the exhalation passage 2. The exhalation introduced into the exhalation passage 2 through the blowing tube 1 is discharged to the outside through a discharge port of a second end of the exhalation passage 2. A collection hole 3 for supplying the part of the exhalation introduced into the exhalation passage 2 to an alcohol sensor 5 is formed in the exhalation passage 2. Most of the exhalation introduced into the exhalation passage 2 is directly discharged to the outside through the discharge port, and a part of the exhalation is supplied to the alcohol sensor 5 through the collection hole 3. A suction pump 4 for introducing a sample gas to the alcohol sensor 5 is mounted on a rear end of the alcohol sensor 5.

However, it is known that droplets are mixed in the exhalation and viruses do not die in the droplets within 3 to 24 hours. Therefore, in a breathalyzer that is used by multiple subjects, another subject may be infected by droplets remaining in a sampling device of the breathalyzer. In addition, when an exhalation is blown, droplets discharged through the discharge port may splay on a measurer's face. In this manner, the conventional breathalyzer has a problem that there is a high risk of occurrence of a droplet infection between a subject and a measurer due to a structure of the sampling device of the conventional breathalyzer.

Document EP 3 059 572 A1 concerns gas sampling means for use with a gas sampling system. The gas sampling means include at least one length of tube and means to connect at least a first end of said tube to a central unit of a gas sampling system and/or a vacuum. At least part of the tube is formed from a porous material. The porous material is provided with a substantially even draw / suction / vacuum and/or the like through pores of the said porous material along substantially the length of the same.

Document KR 101 873 642 B1 relates to a composite gas sensor, and more particularly, to a composite gas sensor provided with a semiconductor type gas sensor in an optical waveguide of a non-dispersion infrared type gas sensor.

### [Document of Related Art]

Korean Patent Application Publication No. 10-2017-0040890
Korean Patent No. 10-1525204
Korean Patent Application Publication No. 10-2016-0096251
Korean Patent No. 10-0706040

### Disclosure

### Technical Problem

Accordingly, the present disclosure has been made keeping in mind the above problems occurring in the related art, and an objective of the present disclosure is to provide a loop-type sampling device capable of minimizing a droplet infection.

In addition, another objective of the present disclosure is to provide a breathalyzer capable of minimizing a droplet infection.

### Summary of the invention

The invention is set out in the appended set of claims.

### Technical Solution

In order to achieve the objectives, according to the present disclosure, there is provided a loop-type sampling device including: a body formed in a loop shape forming a central opening through which a gas passes, the body having an entrance into which a part of the gas that passes through the central opening is introduced, and the body having an inner passage which is formed in a loop shape and into which a sample gas that is the gas passing through the entrance flows; and a sample gas inlet tube having a first end that is in communication with the inner passage, the sample gas inlet tube having a second end connected to a sample gas analysis device that is provided with a negative pressure providing means capable of suctioning the sample gas that flows into the inner passage.

In addition, the body includes: a first body having a guide surface which is formed on the central opening and which has at least of a portion inclined so as to guide the gas to the entrance, the first body having a first coupling surface formed on an opposite side of the guide surface, and the first body having a protrusion surface which extends from the first coupling surface and which forms a protrusion portion together with the guide surface; and a second body having a second coupling surface coupled to the first coupling surface of the first body, the second body having a curved surface which extends from the second coupling surface and which surrounds the protrusion portion of the first body, and the entrance may be formed between an end of the guide surface and an end of the curved surface, and the inner passage may be formed between the protrusion surface and the curved surface.

In addition, a sealing member may be mounted between the first coupling surface and the second coupling surface.

In addition, a concave portion and a convex portion that are engaged with each other may be formed on the first coupling surface and the second coupling surface.

In addition, a breathalyzer may include: the loop-type sampling device; a carbon dioxide sensor configured to generate an electrical signal according to a carbon dioxide concentration of a sample gas introduced through the sample gas inlet tube; an alcohol sensor configured to generate an electrical signal according to an alcohol concentration of the sample gas introduced through the sample gas inlet tube; and a controller configured to determine an inebriation state on the basis of the electrical signal of the alcohol sensor when the carbon dioxide concentration according to the electrical signal of the carbon dioxide sensor is equal to or more than a predetermined value, wherein the negative pressure providing means may be a suction pump connected to a downstream side of the sample gas inlet tube.

### Advantageous Effects

The loop-type sampling device and the breathalyzer of the present disclosure have an advantage that a droplet infection between a subject and a measurer is capable of being minimized.

In the loop-type sampling device according to the present disclosure, since an exhalation is easy to pass through without hitting the loop-type sampling device and the loop-type sampling device has a structure lowering a possibility of remaining droplets, a possibility of occurrence of a droplet infection between a subject and a measurer during a inebriation testing process is lowered when the loop-type sampling device according to the present disclosure is used.

In addition, in the loop-type sampling device according to the present disclosure, since a distance between a subject and a measurer is capable of being increased by extending a length of the sample gas inlet tube, so that a possibility of occurrence of a droplet infection of the measurer caused by droplets contained in the exhalation of the infected subject is lowered.

### Description of Drawings

FIG. 1 is a conceptual view schematically illustrating a conventional breathalyzer.
FIG. 2 is a front view illustrating a breathalyzer according to an embodiment of the present disclosure.
FIG. 3 is a side view illustrating the breathalyzer illustrated in FIG. 2.
FIG. 4 is a conceptual view schematically illustrating the breathalyzer illustrated in FIG. 2.
FIG. 5 is a loop-type sampling device illustrated in FIG. 2.
FIG. 6 is an exploded perspective view illustrating the loop-type sampling device illustrated in FIG. 5.
FIG. 7 is a cross-sectional view of the loop-type sampling device illustrated in FIG. 5 taken along line A-A.

### Best Mode

Hereinafter, an embodiment of the present disclosure will be described in detail with reference to accompanying drawings. The following embodiment is provided by way of example so as to fully convey the spirit of the present disclosure to those skilled in the art. Accordingly, the present disclosure is not limited to the embodiment disclosed herein and can also be implemented in different forms. In addition, the drawings may be exaggerated in width, length, and thickness of components for descriptive convenience and clarity only. Like components will be denoted by like reference numerals throughout the specification.

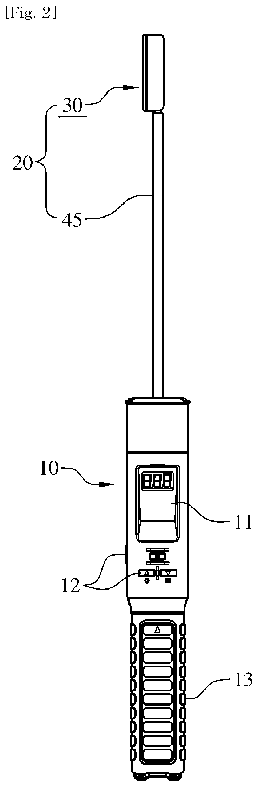

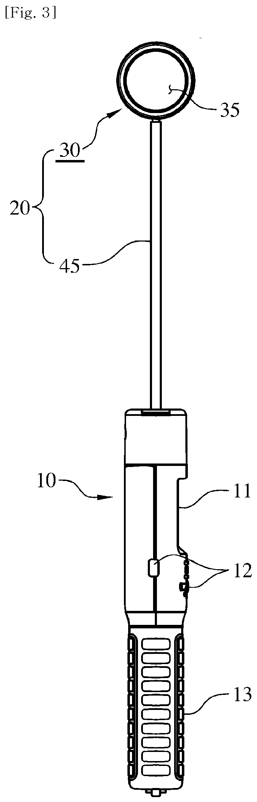

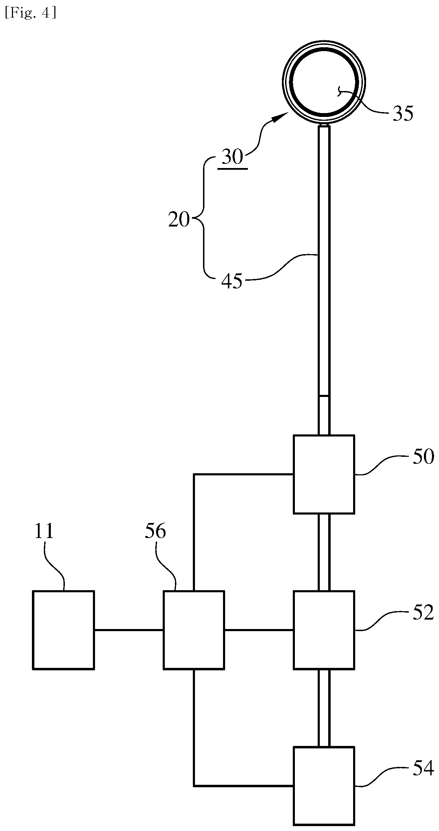

FIG. 2 is a front view illustrating a breathalyzer according to an embodiment of the present disclosure, FIG. 3 is a side view illustrating the breathalyzer illustrated in FIG. 2, and FIG. 4 is a conceptual view schematically illustrating the breathalyzer illustrated in FIG. 2.

As illustrated in FIGS. 2 and 3, a breathalyzer according to an embodiment of the present disclosure includes a housing 10 and a loop-type sampling device 20.

A display portion 11 is mounted on a front surface of an upper portion of the housing 10, and operation buttons 12 are mounted below the display portion 11 and on a side surface of the housing 10. A handle 13 is mounted on a lower portion of the housing 10. The loop-type sampling device 20 mounted on the upper portion of the housing 10 so that the loop-type sampling device 20 is capable of being easily replaced. The display portion 11 of the housing 10 is disposed such that an exhalation blown by a subject does not face a measurer who is looking at the display portion 11. That is, the display portion 11 is disposed such that a direction (a direction perpendicular to the ground in FIG. 3) that a loop-shaped body 30 faces is at a 90 degree angles with a direction that the display portion 11 faces (a right direction in FIG. 3) .

In addition, as illustrated in FIG. 4, the breathalyzer further includes a carbon dioxide sensor 50, an alcohol sensor 52, a suction pump 54, and a controller 56 that are mounted inside the housing 10.

The carbon dioxide sensor 50 is configured to generate an electrical signal according to a carbon dioxide concentration of a sample gas that is collected through the loop-type sampling device 20. A conventional infrared-type gas sensor and so on may be used as the carbon dioxide sensor 50.

The alcohol sensor 52 is configured to generate an electrical signal according to an alcohol concentration of the sample gas passing through the carbon dioxide sensor 50. A conventional semiconductor-type gas sensor, an electrochemical-type gas sensor, and so on may be used as the alcohol sensor 52.

The suction pump 54 serves to introduce the sample gas into inner portions of the carbon dioxide sensor 50 and the alcohol sensor 52 when the suction pump 54 is operated. A diaphragm pump or a solenoid pump may be used as the suction pump 54. In the present disclosure, an upstream side and a downstream side is defined on the basis of a direction where the sample gas flows when the suction pump 54 is operated.

In the embodiment illustrated in FIG. 4, an arrangement order is illustrated that the carbon dioxide sensor 50, the alcohol sensor 52, and the suction pump 54 are sequentially disposed, but the suction pump 54 may be disposed at an upstream side of the alcohol sensor 52.

The controller 56 serves to control the carbon dioxide sensor 50, the alcohol sensor 52, and the suction pump 54. In addition, the controller 56 is also configured such that the controller 56 receives the electrical signal from the carbon dioxide sensor 50 and calculates the carbon dioxide concentration, and receives the electrical signal from the alcohol sensor 52, calculates the alcohol concentration, and transmits a result to the display portion 11.

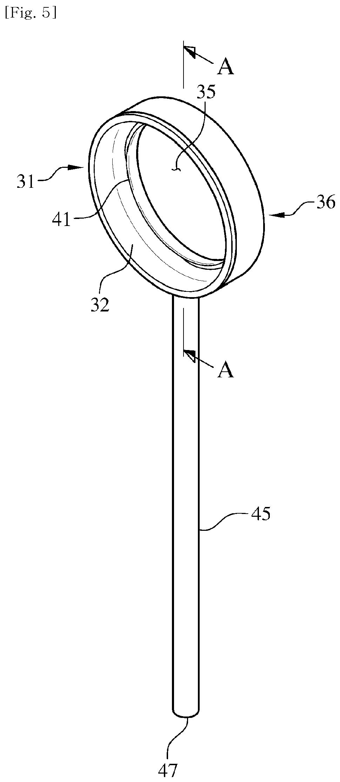

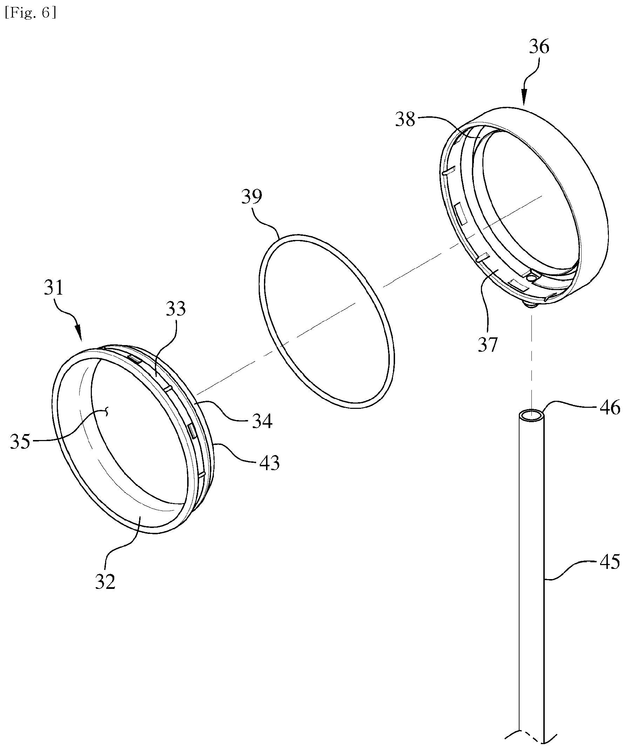

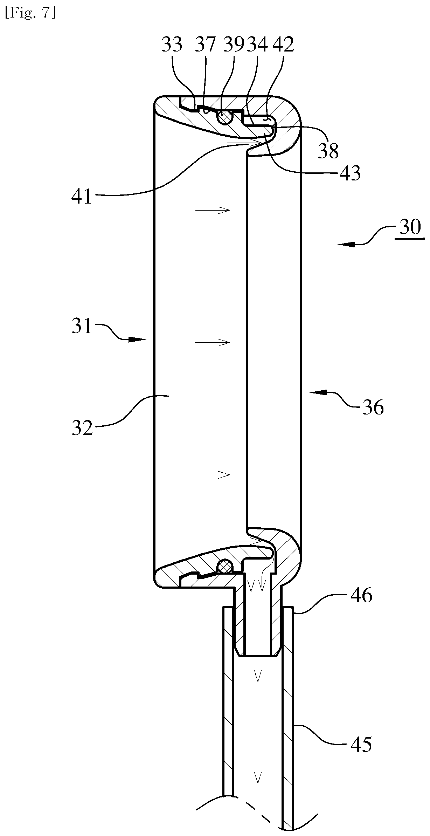

FIG. 5 is a loop-type sampling device illustrated in FIG. 2, FIG. 6 is an exploded perspective view illustrating the loop-type sampling device illustrated in FIG. 5, and FIG. 7 is a cross-sectional view of the loop-type sampling device illustrated in FIG. 5 taken along line A-A.

As illustrated in FIGS. 5 to 7, the loop-type sampling device 20 includes the loop-shaped body 30, and includes a sample gas inlet tube 45 having a first end 46 connected to the body 30 and having a second end 47 coupled to the housing 10.

The body 30 is formed in a loop shape forming a central opening 35 through which air containing an exhalation of a subject such as air around a driver seat of a vehicle passes or an exhalation blown by the subject passes. An entrance 41 where a part of a gas that passes through the central opening 35 is introduced is formed on the central opening 35 of the body 30. In addition, an inner passage 42 through which the sample gas that is a gas introduced through the entrance 41 is formed. The inner passage 42 is formed in an annular loop shape.

The body 30 includes a first body 31 and a second body 36 that are generally formed in annular shapes. The first body 31 includes a guide surface 32 formed on the central opening 35, a first coupling surface 33 formed on an opposite side of the guide surface 32, and a protrusion surface 34 which extends from the first coupling surface 33 and which forms a protrusion portion 43 together with the guide surface 32. At least a portion of the guide surface 32 is inclined so as to guide a gas to the entrance 41. A concave portion and a convex portion are formed on the first coupling surface 33.

The second body 36 includes a second coupling surface 37 coupled to the first coupling surface 33, and includes a curved surface 38 which extends from the second coupling surface 37 and which surrounds the protrusion portion 43 of the first body 31. A concave portion and a convex portion that are corresponding to the concave portion and the convex portion of the first coupling surface 33 are formed on the second coupling surface 37.

When the first body 31 and the second body 36 having the shapes as described above are assembled such that the first coupling surface 33 is in contact with the second coupling surface 37, the entrance 41 is formed between an end of the guide surface 32 and an end of the curved surface 38. In addition, the inner passage 42 is formed between the protrusion surface 34 and the curved surface 38.

The sample gas inlet tube 45 serves to transmit the sample gas flowing through the inner passage 42 to the carbon dioxide sensor 50. A first end of the sample gas inlet tube 45 is in communication with the inner passage 42 of the body 30 from a lower end portion of the body 30, and a second end of the sample gas inlet tube 45 is fitted to the housing 10.

A sealing member 39 is mounted between the first coupling surface 33 and the second coupling surface 37, thereby serving to prevent the sample gas that flows into the inner passage 42 from leaking to a gap between the first coupling surface 33 and the second coupling surface 37. The sealing member 39 may be an O-ring formed of a material having an elasticity. The sealing member 39 may be fitted to a recessed groove formed in the first coupling surface 33 and/or the second coupling surface 37.

Hereinafter, an operation of the breathalyzer according to an embodiment of the present disclosure will be described.

By using a window at the driver seat on which the subject is seated, the body 30 of the loop-type sampling device 20 is disposed around the subject, and then the breathalyzer is operated by using the operation buttons 12 of the housing 10. Then, a warm-up of the carbon dioxide sensor 50 is started, and the suction pump 54 is operated.

When the suction pump 54 is operated, a pressure inside the sample gas inlet tube 45 is lowered, and air around the subject is introduced into the inner passage 42 through the entrance 41 of the loop-type sampling device 20. After the introduced sample gas passes through the sample gas inlet tube 45, the introduced sample gas is introduced into the inner portion of the carbon dioxide sensor 50. In the carbon dioxide sensor 50, an electrical signal according to a carbon dioxide concentration of the sample gas is generated. This electrical signal is transmitted to the controller 56 of the breathalyzer, and the controller 56 calculates the carbon dioxide concentration on the basis of the electrical signal.

When the calculated carbon dioxide concentration is equal to or more than a reference value that is a 2000 ppm as an example, and when it is confirmed that a driver does not perform an evasive action such as ventilating an inside of the vehicle or minimizing breathing before a measurement, the alcohol sensor 52 generates the electrical signal according to the alcohol concentration of the introduced sample gas. When an electrochemical-type gas sensor is used as the alcohol sensor 52, a sensitivity of the alcohol sensor 52 may be increased by operating a solenoid pump two or more times.

This electrical signal is transmitted to the controller 56, and the controller 56 measure the alcohol concentration on the basis of the electrical signal. Then, when the alcohol concentration is equal to or more than a predetermined value that is 3 ppm as an example, the controller 56 determines that the subject is inebriated, and the controller 56 sends a message through the display portion 11 to the measurer.

When the calculated carbon dioxide concentration is less than the reference value or the subject does not agree with a test result, the subject directly blows an exhalation to the body 30 of the loop-type sampling device 20, and the alcohol concentration may be measured again.

### [Description of Reference Numerals]

- 10:: housing
- 11:: display portion
- 12:: operation button
- 13:: handle
- 20:: loop-type sampling device
- 30:: body
- 31: first body
- 32:: guide surface
- 33:: first coupling surface
- 34:: protrusion surface
- 35:: central opening
- 36:: second body
- 37:: second coupling surface
- 38:: curved surface
- 39:: sealing member
- 41:: entrance
- 42:: inner passage
- 45:: sample gas inlet tube

- 50:: carbon dioxide sensor
- 52: alcohol sensor
- 54:: suction pump
- 56:: controller

## Claims

1. A loop-type exhalation sampling device (20) comprising:
a body (30) formed in a loop shape forming a central opening (35) configured so that a gas can pass through, the body (30) having an entrance (41) configured so that a part of the gas that passes through the central opening (35) is introduced into the entrance (41), and the body (30) having an inner passage (42) which is formed in a loop shape and is configured so that a sample gas that is the gas passing through the entrance (41) flows into the inner passage (42); and
a sample gas inlet tube (45) having a first end that is in communication with the inner passage (42), the sample gas inlet tube (45) having a second end connected to a sample gas analysis device that is provided with a negative pressure providing means capable of suctioning the sample gas that flows into the inner passage (42),
whereinthe body (30) comprises:
a first body (31) having a guide surface (32) which is formed on the central opening (35) and which has at least of a portion inclined so as to guide the gas to the entrance (41), the first body (31) having a first coupling surface (33) formed on an opposite side of the guide surface (32), and the first body (31) having a protrusion surface (34) which extends from the first coupling surface (33) and which forms a protrusion portion together with the guide surface (32); and
a second body (36) having a second coupling surface (37) coupled to the first coupling surface (33) of the first body (31), the second body (36) having a curved surface (38) which extends from the second coupling surface (37) and which surrounds the protrusion portion of the first body (31), and
the entrance (41) is formed between an end of the guide surface (32) and an end of the curved surface (38), and the inner passage (42) is formed between the protrusion surface (34) and the curved surface (38).

2. The loop-type exhalation sampling device (20) of claim 1, wherein a sealing member (39) is mounted between the first coupling surface (33) and the second coupling surface (37).

3. The loop-type exhalation sampling device (20) of claim 1, wherein a concave portion and a convex portion that are engaged with each other are formed on the first coupling surface (33) and the second coupling surface (37).

4. A breathalyzer comprising:
the loop-type exhalation sampling device (20) of any one of claims 1 to 3;
a carbon dioxide sensor (50) configured to generate an electrical signal according to a carbon dioxide concentration of a sample gas introduced through the sample gas inlet tube (45) ;
an alcohol sensor (52) configured to generate an electrical signal according to an alcohol concentration of the sample gas introduced through the sample gas inlet tube (45); and
a controller (56) configured to determine an inebriation state on the basis of the electrical signal of the alcohol sensor when the carbon dioxide concentration according to the electrical signal of the carbon dioxide sensor (50) is equal to or more than a predetermined value,
wherein the negative pressure providing means is a suction pump connected to a downstream side of the sample gas inlet tube (45).

## Patentansprüche

1. Schleifenartige Ausatmungsprobenentnahmevorrichtung (20), umfassend:
einen Körper (30), der in einer Schleifenform ausgebildet ist und eine zentrale Öffnung (35) bildet, die so konfiguriert ist, dass ein Gas hindurchtreten kann, wobei der Körper (30) einen Eingang (41) aufweist, der so konfiguriert ist, dass ein Teil des Gases, das durch die zentrale Öffnung (35) hindurchtritt, in den Eingang (41) eingeführt wird, und wobei der Körper (30) einen inneren Durchgang (42) aufweist, der in einer Schleifenform ausgebildet und so konfiguriert ist, dass ein Probengas, das das Gas ist, das durch den Eingang (41) hindurchtritt, in den inneren Durchgang (42) fließt; und
ein Probengaseinlassrohr (45) mit einem ersten Ende, das mit dem inneren Durchgang (42) in Verbindung steht, wobei das Probengaseinlassrohr (45) ein zweites Ende aufweist, das mit einer Probengasanalysevorrichtung verbunden ist, die mit einer Unterdruckerzeugungseinrichtung versehen ist, die in der Lage ist, das in den inneren Durchgang (42) strömende Probengas anzusaugen,
wobei der Körper (30) umfasst:
einen ersten Körper (31) mit einer Führungsfläche (32), die an der zentralen Öffnung (35) ausgebildet ist und die zumindest einen Abschnitt aufweist, der geneigt ist, um das Gas zum Eingang (41) zu leiten, wobei der erste Körper (31) eine erste Kopplungsfläche (33) aufweist, die an einer gegenüberliegenden Seite der Führungsfläche (32) ausgebildet ist, und der erste Körper (31) eine Vorsprungsfläche (34) aufweist, die sich von der ersten Kopplungsfläche (33) erstreckt und die zusammen mit der Führungsfläche (32) einen Vorsprungsabschnitt bildet; und
einen zweiten Körper (36) mit einer zweiten Kopplungsfläche (37), die mit der ersten Kopplungsfläche (33) des ersten Körpers (31) gekoppelt ist, wobei der zweite Körper (36) eine gekrümmte Fläche (38) aufweist, die sich von der zweiten Kopplungsfläche (37) aus erstreckt und die den Vorsprungsabschnitt des ersten Körpers (31) umgibt, und
der Eingang (41) zwischen einem Ende der Führungsfläche (32) und einem Ende der gekrümmten Fläche (38) ausgebildet ist, und der innere Durchgang (42) zwischen der Vorsprungsfläche (34) und der gekrümmten Fläche (38) ausgebildet ist.

2. Schleifenartige Ausatmungsprobenentnahmevorrichtung (20) nach Anspruch 1, wobei ein Dichtungselement (39) zwischen der ersten Kupplungsfläche (33) und der zweiten Kupplungsfläche (37) angebracht ist.

3. Schleifenartige Ausatmungsprobenentnahmevorrichtung (20) nach Anspruch 1, wobei ein konkaver Abschnitt und ein konvexer Abschnitt, die miteinander in Eingriff stehen, auf der ersten Kupplungsfläche (33) und der zweiten Kupplungsfläche (37) ausgebildet sind.

4. Atemanalysator, umfassend:
die schleifenartige Ausatmungsprobenentnahmevorrichtung (20) nach einem der Ansprüche 1 bis 3;
einen Kohlenstoffdioxidsensor (50), der so konfiguriert ist, dass er ein elektrisches Signal in Abhängigkeit von der Kohlenstoffdioxidkonzentration eines durch das Probengaseinlassrohr (45) eingeführten Probengases erzeugt;
einen Alkoholsensor (52), der so konfiguriert ist, dass er ein elektrisches Signal in Abhängigkeit von der Alkoholkonzentration des durch das Probengaseinlassrohr (45) eingeleiteten Probengases erzeugt; und
eine Steuerung (56), die so konfiguriert ist, dass sie auf der Grundlage des elektrischen Signals des Alkoholsensors einen Trunkenheitszustand bestimmt, wenn die Kohlenstoffdioxidkonzentration gemäß dem elektrischen Signal des Kohlenstoffdioxidsensors (50) gleich oder größer als ein vorbestimmter Wert ist, wobei die Unterdruckerzeugungseinrichtung eine Saugpumpe ist, die mit einer stromabwärtigen Seite des Probengaseinlassrohrs (45) verbunden ist.

## Revendications

1. Dispositif d'échantillonnage de l'exhalation de type boucle (20) comprenant:
un corps (30) en forme de boucle formant une ouverture centrale (35) configurée pour permettre le passage d'un gaz, le corps (30) ayant une entrée (41) configurée pour qu'une partie du gaz qui passe par l'ouverture centrale (35) soit introduite dans l'entrée (41), et le corps (30) ayant un passage intérieur (42) en forme de boucle et configuré pour qu'un échantillon de gaz qui est le gaz passant par l'entrée (41) s'écoule dans le passage intérieur (42); et
un tube d'entrée de gaz échantillon (45) ayant une première extrémité en communication avec le passage intérieur (42), le tube d'entrée de gaz échantillon (45) ayant une deuxième extrémité reliée à un dispositif d'analyse de gaz échantillon doté d'un moyen de pression négative capable d'aspirer le gaz échantillon qui s'écoule dans le passage intérieur (42),
où le corps (30) comprend:
un premier corps (31) ayant une surface de guidage (32) qui est formée sur l'ouverture centrale (35) et qui a au moins une partie inclinée de manière à guider le gaz vers l'entrée (41), le premier corps (31) ayant une première surface de couplage (33) formée sur un côté opposé de la surface de guidage (32), et le premier corps (31) ayant une surface de saillie (34) qui s'étend à partir de la première surface de couplage (33) et qui forme une partie de saillie avec la surface de guidage (32);
et
un deuxième corps (36) ayant une deuxième surface de couplage (37) couplée à la première surface de couplage (33) du premier corps (31), le deuxième corps (36) ayant une surface incurvée (38) qui s'étend à partir de la deuxième surface de couplage (37) et qui entoure la partie en saillie du premier corps (31), et
l'entrée (41) est formée entre une extrémité de la surface de guidage (32) et une extrémité de la surface incurvée (38), et le passage intérieur (42) est formé entre la surface de protubérance (34) et la surface incurvée (38).

2. Dispositif d'échantillonnage de l'exhalation de type boucle (20) selon la revendication 1, dans lequel un élément d'étanchéité (39) est monté entre la première surface d'accouplement (33) et la seconde surface d'accouplement (37).

3. Dispositif d'échantillonnage de l'exhalation de type boucle (20) selon la revendication 1, dans lequel une partie concave et une partie convexe engagées l'une dans l'autre sont formées sur la première surface d'accouplement (33) et la seconde surface d'accouplement (37).

4. Éthylomètre comprenant
le dispositif d'échantillonnage de l'exhalation de type boucle (20) de l'une quelconque des revendications 1 à 3;
un capteur de dioxyde de carbone (50) configuré pour générer un signal électrique en fonction de la concentration en dioxyde de carbone d'un échantillon de gaz introduit par le tube d'entrée de gaz échantillon (45);
un capteur d'alcool (52) configuré pour générer un signal électrique en fonction de la concentration en alcool de l'échantillon de gaz introduit par le tube d'entrée de gaz l'échantillon (45); et
un contrôleur (56) configuré pour déterminer un état d'ébriété sur la base du signal électrique du capteur d'alcool lorsque la concentration de dioxyde de carbone selon le signal électrique du capteur de dioxyde de carbone (50) est égale ou supérieure à une valeur prédéterminée,
dans lequel le moyen de fourniture de pression négative est une pompe d'aspiration connectée à un côté aval du tube d'entrée du gaz d'échantillonnage (45).
